(19) Europäisches Patentamt
European Patent Office
Office européen des brevets

(11) Veröffentlichungsnummer: **0 031 795**
**A1**

(12) # EUROPÄISCHE PATENTANMELDUNG

(21) Anmeldenummer: **80810392.3**

(22) Anmeldetag: **15.12.80**

(51) Int. Cl.³: **C 07 D 213/89**, C 07 D 237/18, A 01 N 43/40, A 01 N 43/58, B 27 K 3/34, C 08 K 5/34, C 11 D 3/28, C 11 D 9/50, D 06 M 13/34

(30) Priorität: **21.12.79 CH 11401/79**

(43) Veröffentlichungstag der Anmeldung: **08.07.81 Patentblatt 81/27**

(84) Benannte Vertragsstaaten: **CH DE FR GB IT LI**

(71) Anmelder: **CIBA-GEIGY AG, Patentabteilung Postfach, CH-4002 Basel (CH)**

(72) Erfinder: **Muntwyler, René, Dr., Hutmattweg 6, CH-4114 Hofstetten (CH)**
Erfinder: **Menasse, Raphael, Dr., Mittelweg 19, CH-4142 Münchenstein (CH)**

(54) **Neue Hydroxyphenylketone.**

(57) Hydroxyphenylketone der Formel

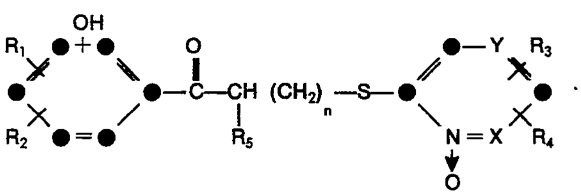

worin n 0, 1, 2 oder 3, X C oder N und Y C, N oder N→O bedeuten, wobei der Heterocyclus höchstens 2 N-Atome als Ringglieder enthält, $R_1$ und $R_2$ jeweils H, F, Cl, Br, $NO_2$, $CH_3$ oder $OCH_3$, $R_3$, H, Cl, Br, $NO_2$, CN, $CH_3$, $OCH_3$, $NH_2$ oder $NHCOCH_3$, $R_4$ H, Cl, Br oder $CH_3$ oder gemeinsam mit $R_3$ einen an die Bindung [b] anellierten Benzolring, wenn X für C steht, und $R_5$ Wasserstoff oder eine Gruppe der Formel

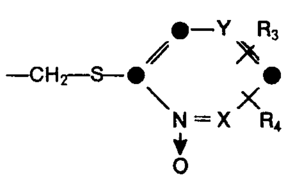

bedeuten, wenn n = 1. Verfahren zur Herstellung dieser Verbindungen werden ebenfalls vorgeschlagen. Die erwähnten Verbindungen können zur Bekämpfung schädlicher Mikroorganismen verwendet werden.

EP 0 031 795 A1

CIBA-GEIGY AG
Basel (Schweiz)

1-12651/+

Neue Hydroxyphenylketone.

Die vorliegende Erfindung betrifft neue Hydroxyphenylketone, die eine 1-Azaaryl-2-thio-1-oxid-Gruppe enthalten, Verfahren zu deren Herstellung, diese enthaltende antimikrobielle Mittel sowie die Verwendung der neuen Verbindungen zur Bekämpfung von Mikroorganismen.

Aus der Literatur sind verschiedene antimikrobiell wirksame Pyridin-N-oxide bekannt, die in 2-Stellung eine Thiolgruppe, oder eine gegebenenfalls weiter substituierte Alkyl-,Aralkyl- oder Arylthiogruppe enthalten. Siehe dazu etwa die U.S. Patentschriften 3,295,946 und 4,048,181 sowie die deutsche Offenlegungsschrift 21 65 752.

Aus der U.S. Patentschrift 3,772,307 sind unter anderem Pyridyl-N-oxid-2-thio-phenylketone bekannt, die als fungizide und bakterizide Wirkstoffe verwendet werden können.

Es wurde nun überraschenderweise gefunden, dass eine bestimmte Gruppe von 1-Azaaryl-1-oxid-2-thio-hydroxyphenylketonen eine besonders gute antimikrobielle Wirkung gegen grampositive und gramnegative Bakterien, Pilze und Hefen aufweisen und dabei die genannten bekannten konstitutionsähnlichen Verbindungen in der Stärke dieser Wirkung übertreffen und gleichzeitig ein sehr breites Wirkungsspektrum besitzen. Ausserdem weisen die neuen erfindungsgemässen Verbindungen eine geringere Toxizität auf als vergleichbare Verbindungen.

Die erfindungsgemässen Hydroxyphenylketone entsprechen der Formel

BAD ORIGINAL

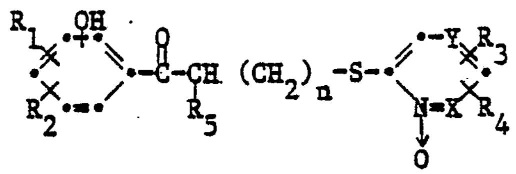 , (1)

worin n die Zahl 0, 1, 2 oder 3 darstellt, wobei die Hydroxylgruppe nur dann in 3-Stellung stehen kann, wenn n 0 oder 1 ist, X ein Kohlenstoff- oder Stickstoffatom, Y ein Kohlenstoff- oder Stickstoffatom oder eine Gruppe $\rangle N \rightarrow O$ bedeuten, wobei der heterocyclische Ring jedoch höchstens 2 Stickstoffatome als Ringglieder enthalten kann, $R_1$ und $R_2$ unabhängig voneinander Wasserstoff, Fluor, Chlor, Brom, Nitro, Methyl oder Methoxy, $R_3$ Wasserstoff, Chlor, Brom, Nitro, Cyano, Methyl, Methoxy, Amino oder $-NHCOCH_3$, $R_4$ Wasserstoff, Chlor, Brom oder Methyl oder $R_3$ und $R_4$ für den Fall, dass X für ein Kohlenstoffatom steht, gemeinsam die Ergänzung zu einem an Bindung [b] des heterocyclischen Ringes anellierten Benzolring und $R_5$ Wasserstoff oder für den Fall, dass n 1 ist, eine Gruppe der Formel

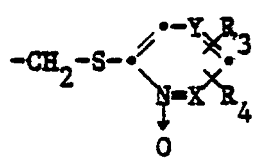 (1a)

bedeuten, wobei $R_3$, $R_4$, X und Y wie oben definiert sind.

Für den Fall, dass $R_3$ und $R_4$ zusammen einen in [b] anellierten Benzolring bedeuten, ergibt sich eine heterocyclische Gruppe der Formel

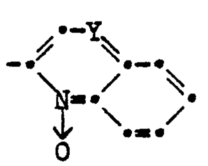 ,

BAD ORIGINAL

worin Y ein Kohlenstoffatom, ein Stickstoffatom oder die Gruppe $\rangle N \rightarrow O$ bedeutet.

Hervorzuheben im Rahmen der Verbindungen der Formel (1) sind die Verbindungen der Formel

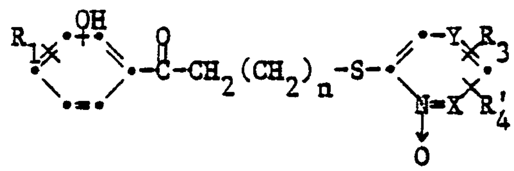
,(2)

worin n, X, Y, $R_1$ und $R_3$ wie in Formel 1 definiert sind und $R_4'$ Wasserstoff oder für den Fall, dass X für ein Kohlenstoffatom steht, gemeinsam mit $R_3$ die Ergänzung zu einem an Bindung [b] des heterocyclischen Ringes anellierten Benzolring bedeutet, sowie der Formel

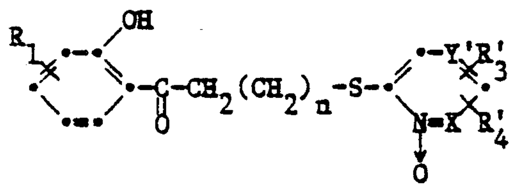
,(3)

worin n, $R_1$ und X wie in Formel 2 definiert sind und Y' ein Kohlenstoff- oder Stickstoffatom, $R_3'$ Wasserstoff, Chlor, Brom oder Methyl und $R_4'$ Wasserstoff oder $R_3'$ und $R_4'$ für den Fall, dass X für ein Kohlenstoffatom steht, gemeinsam die Ergänzung zu einem an Bindung [b] des heterocyclischen Ringes anellierten Benzolring bedeuten.

Von besonderer praktischer Bedeutung sind die Verbindungen der Formel

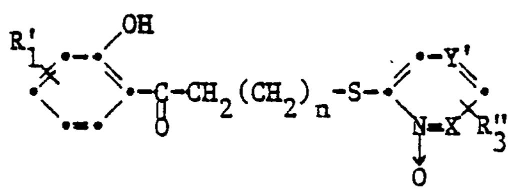
,(4)

worin n die Zahl 0, 1, 2 oder 3, X und Y' unabhängig voneinander ein Kohlenstoffatom oder ein Stickstoffatom, $R_1'$ Wasserstoff, Chlor, Brom, Nitro oder Methyl und $R_3''$ Wasserstoff, Chlor, Brom oder Methyl be-

BAD ORIGINAL

deuten, insbesondere jene der Formel (4), worin $R_1'$ Wasserstoff, Chlor oder Methyl und $R_3''$ Wasserstoff, Chlor oder Methyl bedeuten.

Besondere Erwähnung verdienen auch die Verbindungen der Formel

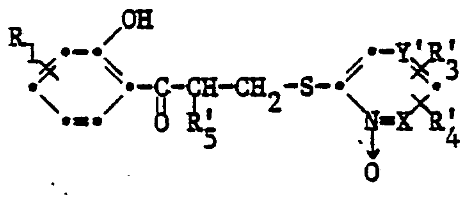
,(5)

worin $R_1$ und X wie in Formel 1 definiert sind und Y' ein Kohlenstoff- oder Stickstoffatom, $R_3'$ Wasserstoff, Chlor, Brom oder Methyl und $R_4'$ Wasserstoff oder $R_3'$ und $R_4'$ für den Fall, dass X für ein Kohlenstoffatom steht, gemeinsam die Ergänzung zu einem an Bindung [b] des heterocyclischen Ringes anellierten Benzolring und $R_5'$ Wasserstoff oder eine Gruppe der Formel

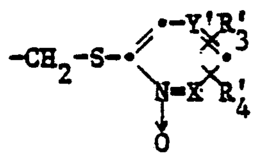
(5a)

bedeuten, wobei $R_3'$, $R_4'$, X und Y' wie oben definiert sind, insbesondere jene der Formel

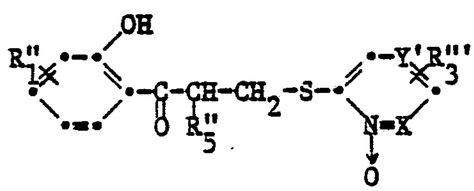
,(6)

worin X und Y' wie in Formel 5 definiert sind und $R_1''$ Wasserstoff, Chlor oder Methyl, $R_3'''$ Wasserstoff, Chlor oder Methyl und $R_5''$ Wasserstoff oder eine Gruppe der Formel

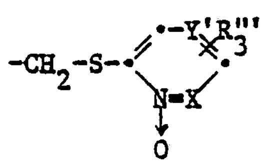
(6a)

BAD ORIGINAL

bedeuten, wobei X, Y' und $R_3'''$ wie oben definiert sind.

Bevorzugt sind unter den Verbindungen der Formeln (1) bis (6) diejenigen, in denen X und Y bzw. Y' jeweils ein Kohlenstoffatom bedeuten.

Besonders bevorzugt sind die Hydroxyphenylketone der Formel

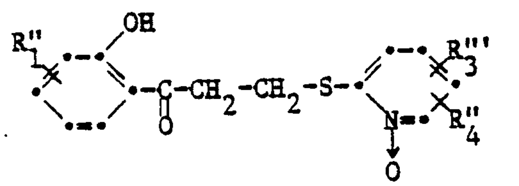 (7) und

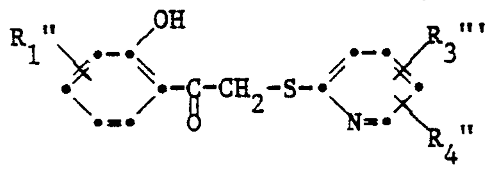 , (7a)

worin $R_1''$, $R_3'''$ und $R_4''$ jeweils unabhängig voneinander Wasserstoff, Chlor oder Methyl bedeuten.

Die neuen Verbindungen der Formeln (1) bis (7) können nach verschiedenen Verfahren hergestellt werden, die ebenfalls Gegenstand der Erfindung sind.

Ein solches Verfahren zur Herstellung von Verbindungen der Formel (1), worin n=1 und damit auch der untergeordneten Formeln (2) bis (7) besteht darin, dass man etwa 1 Moläquivalent einer Mannich-Base der Formel

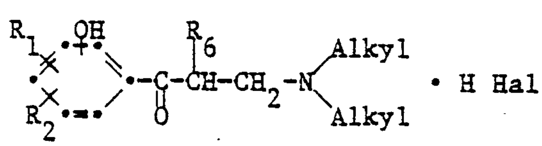 ,(8)

worin $R_1$ und $R_2$ wie in Formel (1) definiert sind und Hal ein Halogenatom und $R_6$ Wasserstoff oder eine Gruppe der Formel

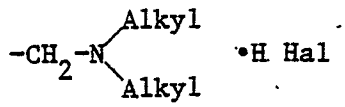 (9)

bedeutet, mit etwa einem Moläquivalent (für den Fall, dass $R_6$ Wasserstoff ist) oder mit etwa 2 Moläquivalent (für den Fall, dass $R_6$ nicht Wasserstoff ist) eines Thiols der Formel

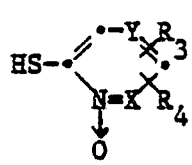 (10)

umsetzt, worin X, Y, $R_3$ und $R_4$ wie in Formel (1) definiert sind.

In den Formeln (8) und (9) bedeutet Hal vorzugsweise Chlor oder Brom, insbesondere Chlor. Die Alkylgruppen in der Mannich-Base haben vorzugsweise 1 bis 6, insbesondere 1 bis 4 C-Atome.

Die Umsetzung der Mannich-Base der Formel (8) mit dem Thiol der Formel (10) wird vorzugweise in einem inerten Lösungsmittel, insbesondere in einem alkoholischen oder wässrig/alkoholischen Medium durchgeführt. Als Alkohole werden insbesondere niedere aliphatische Alkohole mit 1 bis 6, vorzugsweise 1 bis 4 C-Atomen verwendet. Die Umsetzung wird zweckmässig bei erhöhter Temperatur, vorzugsweise beim Siedepunkt des verwendeten Lösungsmittels bzw. Lösungsmittelgemisches durchgeführt. Die Umsetzung kann in Analogie zu der Vorschrift von A.S.Angeloni et al in J. Chem. Soc. [C] 1968, 2295 vorgenommen werden.

Die Mannich-Basen der Formel (8), in denen $R_6$ Wasserstoff bedeutet, können nach dem Schema

BAD ORIGINAL

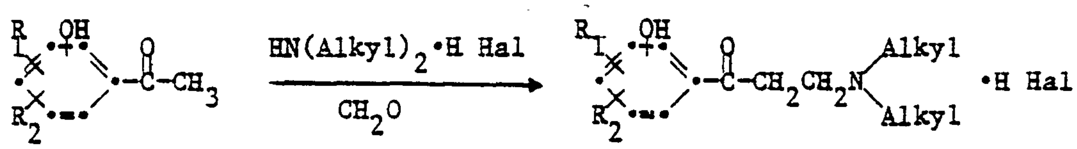


(11) (12)

insbesondere nach den Vorschriften von J.A. Gautier et al, C.R. Acad. Sci. 258, 3731 (1964) und von E.O. Taylor und W.L. Nobles, J.Am. Pharm. Assoc. Sci. Ed. 49, 317 (1960), erhalten werden.

Die Verbindungen der Formel 11 sind bekannt oder können nach bekannten Methoden z.B. aus den entsprechenden Phenolen und Acetylchlorid durch Friedel-Crafts-Reaktion hergestellt werden, gegebenenfalls gefolgt von einer Auftrennung eines unter Umständen anfallenden o-/p-Isomerengemisches.

Die Verbindungen der Formel (8), in denen $R_6$ eine Gruppe der Formel (9) bedeutet, können analog dem vorstehenden Schema aus Verbindungen der Formel (12) durch Mannich-Reaktion mit einem Amin der Formel HN(Alkyl)$_2$•H Hal und Formaldehyd erhalten werden.

Die Thiole der Formel (10) sind bekannt, und zwar aus folgenden Literaturstellen:

| Thiol | | Literaturstelle |
|---|---|---|
| (13) | 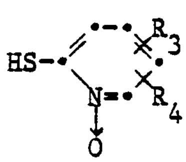 | U.S. Patentschrift 2,745,326<br>" " 4,048,181 |

BAD ORIGINAL

(14) 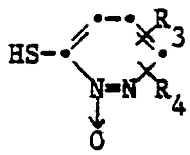 U.S. Patentschrift 4,041,033

(15) 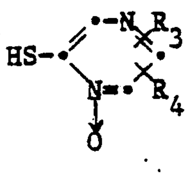 U.S. Patentschrift 4,101,546

(16) 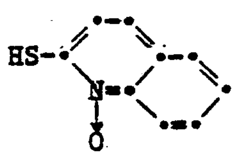 U.S. Patentschrift 3,961,054

(17) 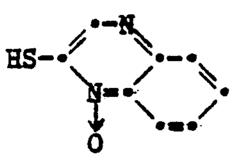 U.S. Patentschrift 3,971,725

(18) 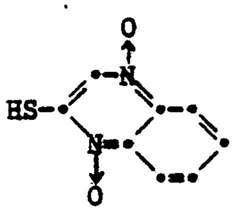 Aus 2-Chlorchinoxalin-di-N-oxid (deutsche Offenlegungsschrift 23 12 730) und NaHS nach bekannten Methoden (U.S. Patentschrift 4,080,329),

oder können in Analogie zu den in den erwähnten Literaturstellen angegebenen Vorschriften leicht erhalten werden.

Weitere Verfahren zur Herstellung von Verbindungen der Formel (1), worin $R_5$ Wasserstoff und n 0 bedeutet, können durch die beiden folgenden Reaktionsgleichungen wiedergegeben werden:

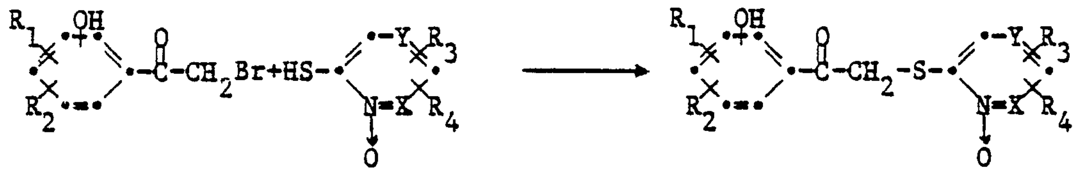

(19) (10) (20)

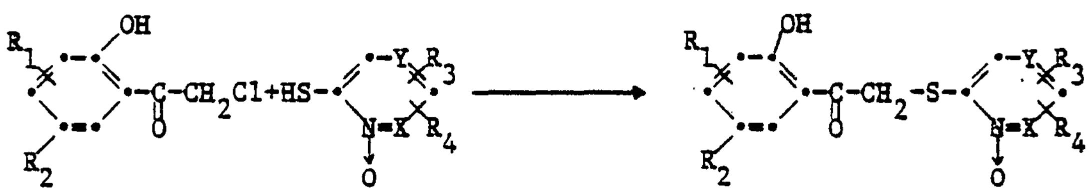

(21) (10) (22)

Die beiden vorstehenden Reaktionen können in Wasser, in Alkoholen, in Dimethylformamid, Dimethylsulfoxid, Acetonitril und ähnlichen Lösungsmitteln, vorzugsweise in Gegenwart einer starken Base, insbesondere eines Alkalimetallalkoholats oder -hydroxyds, beispielsweise bei Raumtemperatur vorgenommen werden. Siehe dazu U.S. Patentschrift 3,772,307.

Die Verbindungen der Formel (19) können nach L.C. Kinz und Ch. K. Ostrum , J. Org. Chem. Soc. 29, 3459 (1964) und die Verbindungen der Formel (21) nach N.M. Cullinane und B.F.R. Edwards, J. Appl. Chem. 9, 133 (1959) erhalten werden.

Ein weiteres Verfahren zur Herstellung von Verbindungen der Formel (1), worin $R_5$ Wasserstoff bedeutet und die Hydroxylgruppe nur dann in 3-Stellung steht, wenn n 0 ist, und damit auch von Verbindungen der entsprechenden untergeordneten Formeln besteht

BAD ORIGINAL

darin, dass man eine Verbindung der Formel

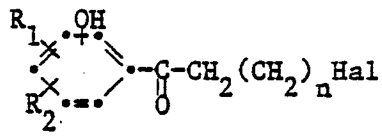

(23)

worin $R_1$, $R_2$ und n wie in Formel (1) definiert sind und Hal Chlor oder Brom bedeutet, wobei Hal nur Brom bedeutet, wenn die Hydroxylgruppe in 3-Stellung steht (nur möglich im Falle n=0), mit einem Thiol der Formel

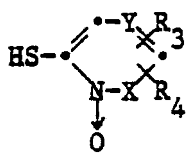

(10)

umsetzt, worin X, Y, $R_3$ und $R_4$ wie in Formel (1) definert sind.

Die Umsetzung erfolgt zweckmässig in einem inerten Lösungsmittel, besonders in Wasser, Alkoholen, Dimethylsulfoxid, Dimethylformamid, Acetonitril oder ähnlichen Lösungsmitteln oder in Mischungen derartiger Lösungsmittel, vorzugsweise in Gegenwart einer starken Base, insbesondere von Alkalimetallalkoholaten oder -hydroxyden. Die Reaktion wird im Falle von n=0 vorzugsweise etwa bei Raumtemperatur, in allen übrigen Fällen vorzugsweise zwischen 60 und 100°C durchgeführt.

Die Verbindungen der Formel (23) können etwa nach folgendem Reaktionsschema erhalten werden:

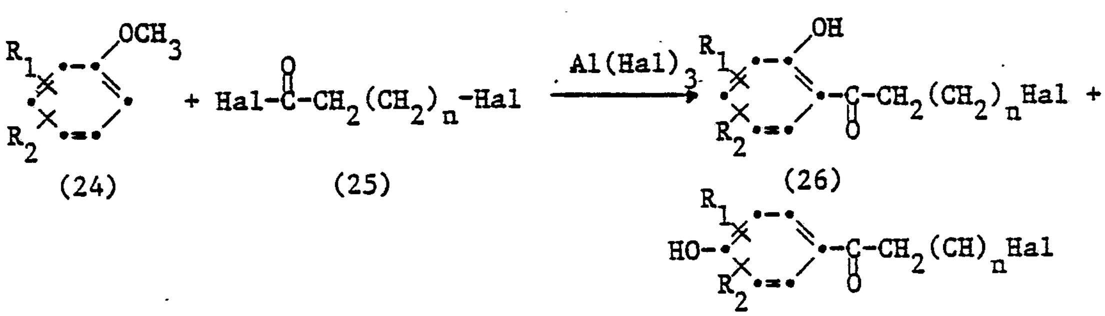


(24) (25) (26) (27)

Hal bedeutet in obigen Formeln Chlor oder Brom. Die Verbindungen der Formeln (24) und (25) sind bekannt.

Die Reaktion (24) + (25) erfolgt in Gegenwart eines Friedel-Crafts-Katalysators, vorzugsweise von Al $Cl_3$ oder Al $Br_3$. Es kann ohne Lösungsmittel gearbeitet werden oder man verwendet ein bei Friedel-Crafts-Reaktionen übliches Lösungsmittel, beispielsweise Schwefelkohlenstoff, Tetrachlorkohlenstoff, Chloroform, Methylenchlorid, Nitromethan, Nitroäthan, Nitrobenzol, Diäthyläther, Petroläther oder Dimethylsulfoxid.

Im Falle von para-substituierten Anisolen der Formel (24) wird nur eine Verbindung (26), im Falle von 2,6-disubstituierten Anisolen (24) oder unsubstituiertem Anisol nur eine Verbindung (27) erhalten. In allen übrigen Fällen erhält man Gemische von Verbindungen der Formeln (26) und (27). Diese kann man nach üblichen Methoden auftrennen.

Die Verbindungen der Formel (23) können auch durch Acylierung der entsprechenden Phenole mit $\omega$-Haloalkansäurehalogeniden, $\omega$-Haloalkansäureanhydriden, $\omega$-Haloalkansäuren und $\omega$-Haloalkansäureestern in Gegenwart eines Kondensationsmittels, beispielsweise Al $Cl_3$, Al $Br_3$, Zn $Cl_2$, $BF_3$ oder Polyphosphorsäure erhalten werden (siehe dazu G.A. Olah, Friedel-Crafts and related Reactions, Vol I (1963), 91).

Den erfindungsgemässen Verbindungen der Formel (1) kommt infolge ihres breiten antimikrobiellen Wirkungsspektrums, das sich insbesondere sowohl auf grampositive als auch gramnegative Bakterien sowie Fungi und Hefen erstreckt, besondere Bedeutung zu. In anwendungstechnischer Hinsicht ist dabei deren Farb- und Geruchlosigkeit sowie die geringe Toxizität von besonderem Vorteil.

BAD ORIGINAL

Die Erfindung betrifft daher auch ein Verfahren zur Bekämpfung von Mikroorganismen auf oder in organischen oder anorganischen Substraten und zum Schützen dieser Substrate vor Mikroorganismen, das dadurch gekennzeichnet ist, dass man mindestens eine Verbindung der Formel (1) besagten Substraten einverleibt oder auf deren Oberfläche aufbringt.

Die vorliegende Erfindung betrifft ferner antimikrobielle Mittel, die durch einen bestimmten Gehalt an einer oder mehreren der in Formel (1) definierten Verbindungen gekennzeichnet sind. Je nach Anwendung des Verfahrens können diese Mittel verschiedene Hilfsmittel und Lösungsmittel enthalten. Die erfindungsgemässen Verbindungen sind gut in organischen Lösungsmitteln und in Treibgasen für Aerosole löslich. Die erfindungsgemässen Mittel können daher solche Lösungsmittel und Treibgase enthalten, wenn ein Aufsprühen oder Auftragen auf Oberflächen erwünscht ist. Natürlich können die erfindungsgemässen Verbindungen auch in Wasser dispergiert oder emulgiert angewendet werden. Weitere Hilfsstoffe, die in erfindungsgemässen Mitteln enthalten sein können, sind in der weiter unten folgenden Zusammenstellung der Anwendungsgebiete der Verbindungen der Formel (1) angeführt. Vorerst seien als derartige Hilfsstoffe erwähnt: Anionaktive Netzmittel, wie Seifen, Benzolsulfonate, kationaktive Netzmittel wie Alkyl-argyl-sulfat, nichtionogene Netzmittel, wie Polyglykoläther und höhere Fettalkohole, Komplexbildner, wie Natriumhexametaphosphat, Duftstoffe, Schaummittel, Emulgiermittel, Weichmacher, Enthärtungsmittel, Lichtschutzmittel, Appreturmittel, Füllstoffe, wie Silikate, Carbonate und/oder Stärkederivate, pharmazeutische und/oder kosmetische Formulierungen.

Die erfindungsgemässen Verbindungen können natürlich auch mit einem festen Träger zu z.B. pulverförmigen Mitteln formuliert werden.

BAD ORIGINAL

Die Verwendung der Verbindungen der Formel (1) und der sie enthaltenden Mittel ist auf sehr breiter Basis möglich, insbesondere zum Schutze von organischen Substraten gegen den Befall durch schädigende und pathogene Mikroorganismen. Die erwähnten Antimikrobika eignen sich demnach unter anderem als Konservierungs- und Desinfektionsmittel für technische Produkte aller Art, sowie zur Desodorierung.

Ein bevorzugtes Anwendungsgebiet der Verbindungen der Formel (1) betrifft die Konservierung von technischen Formulierungen. Von diesen seien beispielsweise aufgezählt: Leime, Bindemittel, Anstrichmittel, Textilhilfsmittel bzw. Veredlungsmittel, Farb- bzw. Druckpasten, Lacke und ähnliche Zubereitungen auf der Basis von organischen und anorganischen Farbstoffen bzw. Pigmenten, auch solche, welche als Beimischung Casein oder andere organische Verbindungen enthalten. Auch Wand- und Deckenanstriche, z.B. solche, die ein eiweisshaltiges Farbbindemittel enthalten, werden durch einen Zusatz von erfindungsgemässen Verbindungen vor dem Befall durch Mikroorganismen geschützt.

Weiters sind als Anwendungsgebiete zu erwähnen: Konservierung von Agrarchemikalien enthaltenden Formulierungen, von Klebstoffen auf Wassergrundlage, z.B. von Tapetenklebstoffen, vor dem Befall durch Mikroorganismen, Verhinderung und Bekämpfung von Bakterien- und Pilzinfektionen in tierischen Oelen, Fetten und Oelemulsionen, wie Schneidölen, Bohrölen. Werden Anstrichmittel, Lacke und Farben mit Hilfe von erfindungsgemässen Verbindungen konserviert, sind auch die daraus hergestellten Anstrichfilme vor dem Befall durch Mikroorganismen geschützt. Auch Weichmacher, Permanentschlichten (z.B. auf Basis von Polyvinylalkohol) oder Stärkeschlichten können ebenfalls geschützt werden. Kunststoffmassen aller Art, z.B. aus Polyamiden, Polycarbonaten, Polyestern, Polyvinylchlorid, Polypropionat oder Polyvinylalkohol werden ebenfalls vorteilhaft durch erfindungsgemässe Verbindungen vor Bakterien- und

BAD ORIGINAL

Pilzbefall geschützt. Bei Verwendung von Weichmachern ist es vorteilhaft, den antimikrobiellen Zusatz dem Kunststoff im Weichmacher gelöst bzw. dispergiert zuzusetzen. Zweckmässig ist für eine möglichst gleichmässige Verteilung im Kunststoff Sorge zu tragen. Die erhaltenen Kunststoffe können für Gebrauchsgegenstände aller Art, bei denen eine Wirksamkeit gegen verschiedenste Keime, wie z.B. Bakterien und Pilze, erwünscht ist, Verwendung finden, so z.B. für Fussmatten, Badezimmervorhänge, Sitzgelegenheiten, Trittroste in Schwimmbädern, Wandbespannungen etc. Durch Einverleibung in entsprechende Wachs- und Bohnermassen erhält man Fussboden- und Möbelpflegemittel mit desinfizierender Wirkung.

Diesen genannten Formulierungen wird (werden) zur Erzielung des gewünschten Effektes die Wirkstoffverbindung(en) der Formel (1) beigemischt und darin möglichst homogen verteilt. Die Wirkstoffverbindung kann dazu als solche in der entsprechenden Menge oder gelöst oder dispergiert in einem Lösungs- oder Dispersionsmittel, das gegebenenfalls noch weitere Hilfsstoffe enthält, eingesetzt werden. Die Menge an Wirkstoff soll mindestens 10 ppm, bezogen auf das zu schützende Material, betragen, aus praktischen Gründen kommen etwa 100 bis 10'000, vorzugsweise 200 bis 5'000 ppm in Frage.

Ferner können die erfindungsgemässen Verbindungen der Formel (1) zum Schützen verschiedenster Oberflächen vor Bakterien- und Pilzbefall verwendet werden. Hier sei vor allem die Behandlung von Holz (als Rohprodukt), verarbeiteten Gegenständen aus Holz, Holzspänen, Sägemehl, Leder, Häuten und Fellen zu erwähnen. Aber auch Desinfektion und Schutz von Behältern, in denen etwa technische Formulierungen aufbewahrt werden, von Böden und Wänden von Stallungen, Schlachthöfen usw. ist mit Hilfe des erfindungsgemässen Verfahrens möglich. Die erwähnten Gegenstände bzw. Oberflächen werden mit einer den Wirkstoff enthaltenden, wässrigen oder organischen Lösung bzw. Dispersion je nach Gestalt des zu schützenden Gegenstandes besprüht (z.B. mit Hilfe eines Sprays), angestrichen oder getränkt

BAD ORIGINAL

(z.B. Holz, Leder).

Als organische Lösungsmittel seien beispielsweise mit Wasser nicht mischbare Lösungsmittel, insbesondere Petrolfraktionen, aber auch mit Wasser mischbare Lösungsmittel wie niedere Alkohole (z.B. Aethanol, Methanol), Aethylenglykolmonomethyläther oder -monoäthyläther genannt.

Vorzugsweise wird dabei so viel des erfindungsgemässen Mittels angewendet, dass der jeweilige Gegenstand nach der Behandlung etwa 0,01 bis 10 $g/m^2$ an Wirkstoff enthält. Bei der Behandlung von Holz kann vor allem die bei der Lagerung durch verschiedene Fungi hervorgerufene Verfärbung und die Verrottung verhindert bzw. verzögert werden.

Auch in der Zellstoff- und Papierindustrie können die Verbindungen der Formel (1) und das erfindungsgemässe Verfahren angewendet werden. Hier wird damit vor allem die durch Mikroorganismen hervorgerufene Schleimbildung in den zur Papiergewinnung verwendeten Apparaturen verhindert. Zu diesem Zweck wird die entsprechende Wirkstoffverbindung entweder der Papiermasse oder dem Wasserkreislauf in der Papierfabrik zugesetzt. Auch in anderen Industrieanlagen, in denen Beeinträchtigungen durch Mikroorganismen zu erwarten sind, kann das erfindungsgemässe Verfahren in analoger Form angewendet werden. Die Menge an Wirkstoff soll in der Regel mindestens 1 ppm, in der Praxis etwa 10 bis 10'000, vorzugsweise 20 bis 5'000 ppm betragen.

Durch Kombination der erfindungsgemässen Verbindungen mit grenzflächenaktiven, insbesondere waschaktiven Stoffen gelangt man zu Wasch- und Reinigungsmitteln mit ausgezeichneter antibakterieller bzw. antimykotischer Wirkung.

BAD ORIGINAL

Die Reinigunsmittel können in beliebiger, z.B. flüssiger, breiartiger, fester, flockiger oder körniger Form vorliegen. Die Wirkstoffverbindungen können sowohl in anionaktive Verbindungen, wie Seifen und andere Carboxylate (z.B. Alkalisalze höherer Fettsäuren), Abkömmlinge von Schwefelsauerstoffsäuren (z.B. Natriumsalz der Dodecylbenzosulfonsäure, wasserlösliche Salze von Schwefelsäuremonoestern höhermolekularer Alkohole oder ihrer Polyglykoläther, wie etwa löslische Salze von Dodecylalkoholsulfat oder von Dodecylalkoholpolyglykoläthersulfat), Abkömmlinge von Phosphor-Sauerstoffsäuren(z.B. Phosphate), Abkömmlinge mit saurem (elektrophilem) Stickstoff in der hydrophilen Gruppe (z.B. Disulfinsalze), als auch in kationaktive Tenside, wie Amine und ihre Salze (z.B. Lauryldiäthylentriamin), Oniumverbindungen, Aminoxyde oder nichtionogene Tenside, wie Polyhydroxyverbindungen, Tenside auf Mono- oder Polysaccharid-Basis, höhermolekulare Acetylenglykole,Polyglykoläther (z.B. Polyglykoläther höherer Fettalkohole, Polyglykoläther höhermolekularer alkylierter Phenole), bzw. Gemische aus verschiedenartigen Tensiden eingearbeitet werden. Dabei bleibt ihre antimikrobielle Wirksamkeit in vollem Umfange erhalten. Der Wirkstoffgehalt der Reinigungsmittel, bezogen auf das Gewicht dieses Mittels, beträgt im allgemeinen 0,01 bis 20%, meistens 0,01 bis 3%. Wässrige Zubereitungen solcher Reinigungsmittel, welche die Wirkstoffverbindungen enthalten, können z.B. zur antimikrobiellen Ausrüstung von Textilmaterialien verwendet werden; Sie eignen sich ebenfalls als antimikrobielle Reinigungsmittel in der Lebensmittel- und Getränkeindustrie, z.B. in Brauereien, Molkereien, Käsereien, Stallungen und Schlachthöfen.

Im weiteren lassen sich die erfindungsgemässen Verbindungen auch in kosmetische Präparate, wie z.B. Seifen verschiedenster Art, ätherische Oele, Badesalze, Brillantinen, Salben, Gesichtswasser, Haarfärbemittel, Haaröle, Haarwässer, Hautcremes, Hautöle, Kölnisch Wasser, Parfüme, Puder, Schminken, Depilatorien, Sonnenbrandmittel, Zahnpflegemittel usw., einarbeiten, womit diesen Mitteln zusätzlich

BAD ORIGINAL

antimikrobielle und desodorierende Wirkung verliehen wird. Dazu genügt im allgemeinen, bezogen auf das Gesamtgewicht des Mittels, ein Wirkstoffgehalt von 0,001 bis 5%, vorzugsweise von 0,01 bis 3%. Insbesondere eignen sich die erfindungsgemässen Verbindungen als Wirkstoffe in Händedesinfektionsmitteln.

Die erfindungsgemässen Verbindungen können auch zum Desinfizieren und Ausrüsten von Wäsche, z.B. Arztkleidung, Spitalwäsche und zum Desinfizieren von verschiedensten Gegenständen, z.B. im medizinischen Bereich verwendet werden. Diverse Oberflächen, z.B. aus Metall, Kunststoffen, Farbanstriche u.a. können durch erfindungsgemässe Mittel desinfiziert werden. Hier seien Bodenbeläge, Teppichböden, Wände und Einrichtungsgegenstände erwähnt.

Bedeutung kommt den erfindungsgemässen Verbindungen auch bei der Entkeimung von Waschgut und beim Schützen von Waschgut gegen Befall durch Mikroorganismen zu.

Als Waschgut, welches mit erfindungsgemässen Mitteln entkeimt werden kann, kommt vor allem organisches Fasermaterial in Betracht, nähmlich solches natürlicher Herkunft, wie cellulosehaltiges, beispielsweise Baumwolle oder polypeptidhaltiges, zum Beispiel Wolle oder Seide, oder Fasermaterial synthetischer Herkunft, wie solches auf Polyamid-, Polyacrylnitril- oder Polyesterbasis oder Mischungen obengenannter Fasern.

Die erfindungsgemässen Verbindungen verleihen dem damit behandelten Waschgut eine weitgehende Keimfreiheit gegen Staphylokokken-, Coli- und Pseudomonasformen. Ein besonderer Vorteil der erfindungsgemässen Mittel ist dabei, dass die Entkeimung, auch von Peudomonaden, bei tiefen Temperaturen und unter schonenden Bedingungen durchgeführt werden kann, und keine Zusätze von weiteren schädlichen bzw. umweltbelastenden Stoffen nötig sind.

BAD ORIGINAL

Die erfindungsgemässen Verbindungen sind auch gegen die Schweissgeruch erzeugende Bakterienflora sehr wirksam und darum und wegen ihrer geringen Toxizität als desodorierende Mittel für Wäsche oder für kosmetische Mittel geeignet.

Weiters werden die erfindungsgemässen Verbindungen zur konservierenden und desinfizierenden Ausrüstung von Fasern und Textilien verwendet, wobei die Wirkstoffverbindungen auf natürliche und künstliche, vorzugsweise natürliche Fasern, z.B. aus Cellulose, aufgebracht werden und dort eine dauerhafte Wirkung gegen schädliche (auch pathogene) Mikroorganismen, z.B. Pilze und Bakterien, entfalten. Der Zusatz der Verbindungen kann dabei vor, gleichzeitig mit, oder nach einer Behandlung dieser Textilien mit anderen Stoffen, z.B. Farb- oder Druckpasten, Flammfestmitteln, Weichgriffmitteln und anderen Appreturen usw. erfolgen.

Derart behandelte Textilien weisen einen Schutz vor Verrottung, wie sie durch Mikroorganismen bedingt ist, auf.

Die Anwendungsformen der erfindungsgemässen Wirkstoffe können den üblichen Formulierungen entsprechen. Die zum Ausrüsten bzw. zum Schützen von Textilien verwendeten Mittel sollten die erfindungsgemässen Verbindungen in fein verteilter Form enthalten. Zur Anwendung kommen deshalb insbesondere Lösungen, Dispersionen und Emulsionen der Wirkstoffe. Wässrige Dispersionen können beispielsweise aus Pasten oder Konzentraten erhalten werden und flüssig oder als Aerosol angewendet werden.

Die wässrigen Lösungen bzw. Dispersionen enthalten demnach zweckmässig Tenside, beispielsweise anionaktive Verbindungen, wie Seifen und andere Carboxylate (z.B. Alkalisalze höherer Fettsäuren), Abkömmlinge von Schwefel-Sauerstoffsäuren (z.B. Natriumsalz der Dodecylbenzolsulfonsäure, wasserlösliche Salze von Schwefelsäure-

BAD ORIGINAL

monoestern höhermolekularer Alkohole oder ihrer Polyglykoläther, wie etwa lösliche Salze von Dodecylalkohol-sulfat oder von Dodecylalkoholpolyglykolüthersulfat), Abkömmlinge von Phosphor-Sauerstoffsäuren (z.B. Phosphate), Abkömmlinge mit saurem (elektrophilem) Stickstoff in der hydrophilen Gruppe (z.B. Disulfinsalze), kationaktive Tenside, wie Amine und ihre Salze (z.B. Lauryl-diäthylentriamin), Oniumverbindungen, Aminoxyde oder nichtionogene Tenside, wie Polyhydroxyverbindungen, Tenside auf Mono- oder Polysaccharidbasis, höhermolekulare Acetylenglykole, Polyglykoläther (z.B. Polyglykoläther höherer Fettalkohole, Polyglykoläther höhermolekularalkylierter Phenole). Daneben kann die Flotte auch noch übliche Hilfsstoffe, wie wasserlösliche Perborate, Polyphosphate, Carbonate, Silikate, optische Aufheller, Weichmacher, sauer reagierende Salze, wie Ammonium- oder Zinksilikonfluorid, oder gewisse organische Säuren, wie Oxalsäure, ferner Appreturmittel, z.B. solche auf Kunstharzbasis oder Stärke, enthalten.

Die Textilmaterialien können z.B. durch heisse oder kalte Färbe-, Bleich-, Chromierungs- oder Nachbehandlungsbäder mit den Wirkstoffen imprägniert werden, wobei verschiedene Textilausrüstungsverfahren, wie z.B. das Foulard- oder Ausziehverfahren, in Frage kommen.

Die Behandlung erfolgt zweckmässig bei Temperaturen von 10 bis 100°C, beispielsweise bei 10 bis 70°C, vorzugsweise etwa bei Raumtemperatur.

Wegen der guten Löslichkeit in organischen Lösungsmitteln eignen sich die Verbindungen der Formel (1) auch gut zur Applikation aus nicht-wässrigen Medien. Dabei können die auszurüstenden bzw. zu schützenden Materialien einfach mit den Lösungen imprägniert werden.

Als organische Lösungsmittel kommen beispielsweise Trichloräthylen, Methylenchlorid, Kohlenwasserstoffe, Propylenglykol, Methoxyäthanol, Aethoxyäthanol, Dimethylformamid in Frage, denen noch Verteilungsmittel (z.B. Emulgatoren, wie sulfiertes Ricinusöl, Fettalkoholsulfate usw.) und/oder andere Hilfsstoffe zugesetzt werden können.

Der Gehalt an erfindungsgemässen Wirkstoffen kann je nach Anwendungszweck zwischen 0,01 und 50 g, vorzugsweise zwischen 0,1 und 30 g Wirksubstanz pro Liter Behandlungsflüssigkeit liegen.

Die Wirkstoffe können allein oder zusammen mit anderen bekannten antimikrobiellen Textilschutzmitteln angewendet werden.

Als Textilien, die ausgerüstet bzw. geschützt werden, kommen hauptsächlich Fasern natürlicher Herkunft, wie cellulosehaltige, z.B. Baumwolle, oder polypeptidhaltige, z.B. Wolle oder Seide, aber auch synthetischer Herkunft, wie solche auf Polyamid-, Polyacrylnitril- oder Polyesterbasis, oder Mischungen dieser Fasern in Betracht.

Die Menge an auf die Textilmaterialien applizierter Wirkstoffverbindung beträgt vorzugsweise mindestens 100 ppm, bezogen auf das Material.

Meistens werden die Textilien mit einer Menge von 100 bis 10'000, vorzugsweise 300 bis 5'000 ppm an Wirkstoff, bezogen auf das Gewicht der textilen Materialien, ausreichend gegen Pilz- und Bakterienbefall geschützt.

Für Desinfektions- und Konservierungszwecke können die Verbindungen der Formel (1) auch in Kombination mit bekannten antimikrobiellen Mitteln verwendet werden. Als antimikrobielle

BAD ORIGINAL

Mittel, die auf diese Weise in Kombination mit den erfindungsgemässen Verbindungen eingesetzt werden können, kommen beispielsweise solche aus den folgenden Klassen in Frage:

Phenolderivate wie 3,5-Dichlorphenol, 2,5-Dichlorphenol, 3,5-Dibromphenol, 2,5-Dibromphenol, 2,5-(bzw. 3,5-)-Dichlor-4-bromphenol, 3,4,5-Trichlorphenol, 3,4,5-Tribromphenol, Phenylphenol, 4-Chlor-2-phenylphenol, 4-Chlor-2-benzylphenol, Dichlorophen, Hexachlorophen; Aldehyde wie Formaldehyd, Glutaraldehyd, Salicylaldehyd; Alkohole wie Phenoxyäthanol; antimikrobiell wirksame Carbonsäuren und deren Derivate; metallorganische Verbindungen wie Tributylzinnverbindungen; Jodverbindungen wie Jodophore, Jodoniumverbindungen; quaternäre Ammoniumverbindungen wie Benzyldimethyldodecylammoniumchlorid, Dimethyl-didecylammoniumchlorid, Benzyl-di-(2-hydroxyäthyl)-dodecylammoniumchlorid; Sulfonium- und Phosphoniumverbindungen; Mercaptoverbindungen sowie deren Alkali-, Erdalkali- und Schwermetallsalze wie 2-Mercaptopyridin-N-oxid sowie dessen Na- und Zn-Salz, 3-Mercaptopyridazin-2-oxid, 2-Mercapto-chinoxalin-1-oxid, 2-Mercapto-chinoxalin-di-N-oxid, sowie auch die symmetrischen Disulfide der genannten Mercaptoverbindungen; Harnstoffe wie Tribrom- oder Trichlorcarbanilid, Dichlor-trifluormethyl-diphenylharnstoff; Tribromsalicylanilid; 2-Brom-2-nitro-1,3-dihydroxypropan; Dichlorbenzoxazolon; Chlorhexidin; Isothia- und Benzisothiazolonderivate.

Die erfindungsgemässen Verbindungen der Formeln (1) bis (7a) können auch mit Erfolg im Pflanzenschutz verwendet werden. Sie können daher in Pflanzenschutzmitteln als Wirksubstanzen enthalten sein. Die erfindungsgemässen Verbindungen zeigen vor allem Wirkung gegen pflanzenschädigende Fungi, beispielsweise gegen Puccinia-Arten, Botrytis Zinerea und Venturia Inaequalis.

Die nachfolgenden Beispiele dienen der weiteren Erläuterung der Erfindung, ohne sie jedoch darauf zu beschränken. Teile und Prozente

BAD ORIGINAL

sind darin, ebenso wie in der übrigen Beschreibung und in den Patentansprüchen Gewichtsteile bzw. Gewichtsprozent, sofern nichts anderes angegeben.

<u>Beispiel 1</u>: 6,4 g Bis-α,α-(dimethylaminomethyl)-2-hydroxyacetophenon-dihydrochlorid und 5,1 g 2-Mercaptopyridin-1-oxid werden in 20 ml wasserfreiem Aethanol gelöst und 15 Stunden unter Rückfluss erhitzt. Die abgekühlte, klare Lösung wird am Rotationsverdampfer auf das halbe Volumen eingeengt und anschliessend einen Tag bei 5°C stehen gelassen. Die abgeschiedenen Kristalle werden abfiltriert und aus Toluol umkristallisiert. Man erhält so 4,6 g Bis-α,α-(2-pyridylthiomethyl-1-oxid)-2-hydroxyacetophenon der Formel

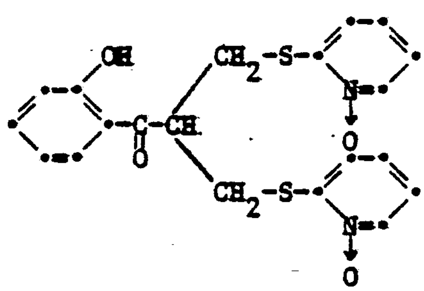

(100)

in Form von schwach bräunlichen Kristallen mit einem Schmelzpunkt von 126-127°C.

Auf analoge Weise unter Verwendung der entsprechenden Mannich-Basen von 3- bzw. 4-Hydroxyacetophenon erhält man die Verbindungen der Formeln

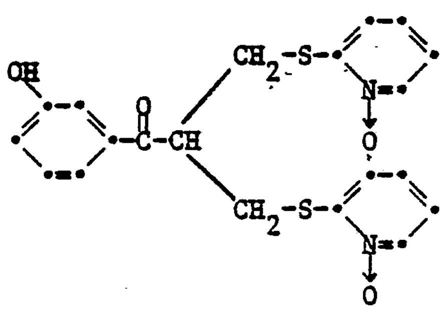

(101) und

BAD ORIGINAL

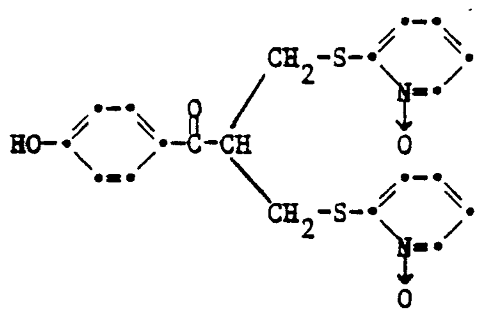

(102)

Die als Ausgangsverbindungen benötigten Mannich-Basen werden folgendermassen erhalten:

45,9 g β-Dimethylamino-2-hydroxy-propiophenonhydrochlorid, 33 g Dimethylaminhydrochlorid und 10 g Paraformaldehyd werden in eine Mischung von 70 ml Aethanol und 0,5 ml konzentrierter Salzsäure eingerührt. Nach 1 1/2 tägigem Rühren unter Rückfluss wird die auf Raumtemperatur abgekühlte Lösung mit 300 ml Aceton versetzt und 15 Stunden bei 5°C stehen gelassen. Die abgeschiedene Substanz wird abgenutscht und im Trockenschrank bei 80°C getrocknet. Man erhält auf diese Weise 33,9 g Bis-α,α-(dimethylaminomethyl)-2-hydroxyacetophenon-dihydrochlorid der Formel

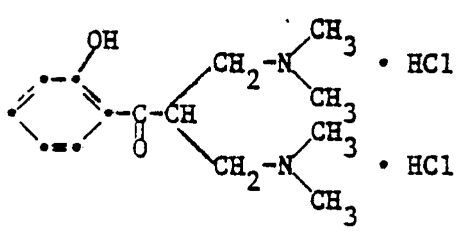

(103)

als farblose Kristalle mit einem Schmelzpunkt von 220-230°C.

Auf analoge Weise unter Verwendung von β-Dimethylamino-3 (bzw. 4)-hydroxy-propiophenon erhält man die Mannich-Basen der Formeln

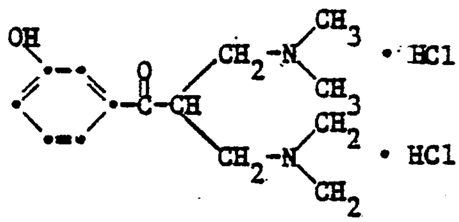

(104) und

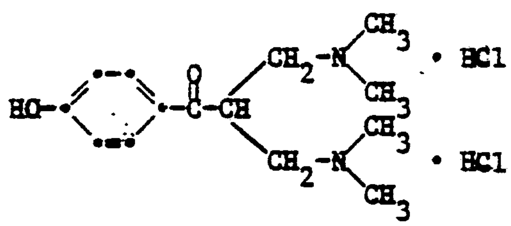

(105)

Die zur Herstellung der Mannich-Basen vom Typ der Formeln (103) bis (105) benötigten β-Dimethylamino-hydroxy-propiophenone werden nach bekannten Methoden hergestellt, z.B. nach J.A. Gautier et al, C.R. Acad. Sci. 258 (1964), 3731, z.B. die in Tabelle 1 angeführten Verbindungen der Formel

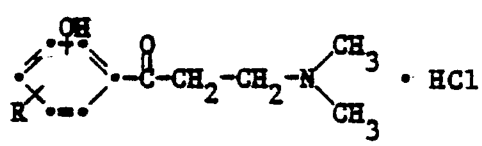

(106)

Tabelle 1

| Verbindung Nr. | Stellung OH | R | Fp (°C) |
|---|---|---|---|
| 107 | 2-OH | H | 176-178 |
| 108 | 3-OH | H | 181-182 |
| 109 | 4-OH | H | 196-198 |
| 110 | 2-OH | 5-Cl | 187-190 |
| 111 | 2-OH | 4-$OCH_3$ | 168-169 |

BAD ORIGINAL

Beispiel 2: 45,9 g β-Dimethylamino-2-hydroxypropiophenonhydrochlorid und 25,4 g 2-Mercaptopyridin-1-oxid werden in 200 ml Methanol gelöst und 15 Stunden lang am Rückfluss erhitzt. Die klare Lösung wird auf 5°C abgekühlt und 15 Stunden bei dieser Temperatur stehen gelassen. Die abgeschiedenen Kristalle (49 g) werden abgenutscht und weisen einen Schmelzpunkt von 159-163°C auf. Nach Umkristallisation aus Chloroform werden 43 g β-(2-Pyridylthio-1-oxid)-2-hydroxypropiophenon der Formel

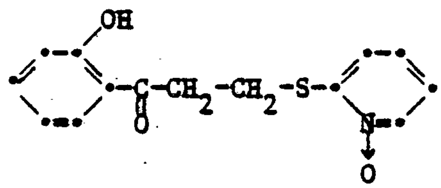

(200)

in Form schwach bräunlicher Kristalle mit einem Schmelzpunkt von 162-164°C erhalten.

Auf analoge Weise werden unter Verwendung der geeigneten Ausgangsprodukte die in Tabelle 2 angeführten Verbindungen der Formel

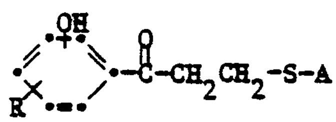

(201)

erhalten:

Tabelle 2

| Verbindung Nr. | Stellung OH | R | A | Fp (°C) |
|---|---|---|---|---|
| 202 | 3-OH | H | 2-Pyridyl-1-oxid | 187-191 |
| 203 | 4-OH | H | 2-Pyridyl-1-oxid | 234-235 |
| 204 | 2-OH | 5-Cl | 2-Pyridyl-1-oxid | 177-179 |

BAD ORIGINAL

| Verbindung Nr. | Stellung OH | R | A | Fp (°C) |
|---|---|---|---|---|
| 205 | 2-OH | H | 3-Pyridazinyl-2-oxid | 109-112 |
| 206 | 2-OH | H | 3-Pyridazinyl-6-chlor -2-oxid | 174-177 |
| 207 | 2-OH | H | 2-Pyrazinyl-1-oxid | |
| 208 | 2-OH | H | 2-Chinolyl-1-oxid | |
| 209 | 2-OH | H | 2-Chinoxalinyl-1-oxid | |
| 210 | 2-OH | H | 2-Chinoxalinyl-1,4-dioxid | |
| 211 | 2-OH | 4-$OCH_3$ | 2-Pyridyl-1-oxid | 145-147 |

Beispiel 3: 12,7 g α-Brom-2-hydroxyacetophenon, 6,35 g 2-Mercaptopyridin-1-oxid und 2,7 g Natriummethylat werden in 100 ml wasserfreiem Aethanol gelöst und 18 Stunden bei Raumtemperatur gerührt; es bildet sich eine dickflüssige Suspension. Diese wird 15 Stunden bei 5°C stehen gelassen und danach wird das unlösliche Material abfiltriert. Der Rückstand wird aus Aethanol umkristallisiert, worauf man 9,5 g α-(2-Pyridylthio-1-oxid)-2-hydroxyacetophenon der Formel

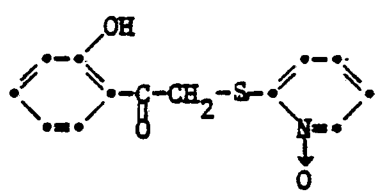

(300)

in Form schwach bräunlicher Kristalle mit einem Schmelzpunkt von 151-153°C erhält.

Auf analoge Weise werden unter Verwendung der entsprechenden Ausgangsmaterialien die in Tabelle 3 angeführten Verbindungen der Formel

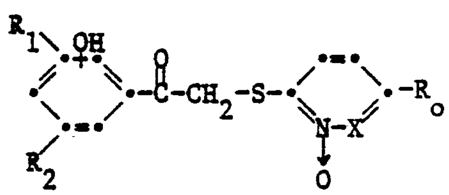

(301)

erhalten.

BAD ORIGINAL

Tabelle 3

| Verbindung Nr. | Stellung OH | $R_o$ | $R_1$ | $R_2$ | X | Fp (°C) |
|---|---|---|---|---|---|---|
| 302 | 2-OH | H | H | Cl | C | 195-197 |
| 303 | 2-OH | H | H | $CH_3$ | C | 180-181 |
| 304 | 2-OH | H | Cl | Cl | C | 177-180 |
| 305 | 3-OH | H | H | H | C | 189 (Zers.) |
| 306 | 4-OH | H | H | H | C | 195 (Zers.) |
| 307 | 2-OH | H | H | H | N | 158-164 |
| 307a | 2-OH | Cl | H | H | N | 207-209 |

Die im vorstehenden Beispiel als Ausgangsprodukte benötigten α-Halogenhydroxyacetophenone werden nach bekannten Methoden hergestellt, z.B. nach L.C. King und G.K. Ostrum, J. Org. Chem. 29, 3459 (1964) (= Methode a) oder nach N.M. Cullinane und B.F.R. Edwards, J. Appl. Chem. 9, 133 (1959) (= Methode b), z.B. die in der folgenden Tabelle 4 angeführten Verbindungen der Formel

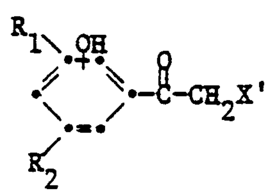

Tabelle 4

| Verbindung Nr. | Stellung OH | $R_1$ | $R_2$ | X' | Methode | Fp (°C) |
|---|---|---|---|---|---|---|
| 308 | 2-OH | H | H | Br | a | 41-43 |
| 309 | 2-OH | Cl | Cl | Cl | b | 132-134 |
| 310 | 2-OH | H | $CH_3$ | Cl | b | 62-63 |
| 311 | 2-OH | H | Cl | Cl | b | 107-110 |
| 312 | 3-OH | H | H | Br | a | dunkles Harz |
| 313 | 4-OH | H | H | Br | a | dunkles Harz |

BAD ORIGINAL

Beispiel 4

20,9 g δ-Chlor-2-hydroxy-5-chlorvalerophenon, 10,2 g 2-Mercaptopyridin-1-oxid und 4,3 g Natriummethylat werden in 150 ml wasserfreiem Aethanol gelöst und 15 Stunden unter Rückfluss erhitzt. Die auf Raumtemperatur abgekühlte Lösung wird auf 500 ml Wasser ausgetragen und 1 Stunde verrührt. Der Feststoff wird abgenutscht und im Trockenschrank bei 60°C getrocknet. Man erhält 26,2 g einer bräunlichen Substanz mit einem Schmelzpunkt von 192-199°C. Diese wird in heissem Chloroform verrührt, 15 Stunden bei 5°C stehen gelassen und danach der unlösliche Feststoff abfiltriert und getrocknet. Man erhält so 22 g δ-(2-Pyridylthio-1-oxid)-2-hydroxy-5-chlor-valerophenon der Formel

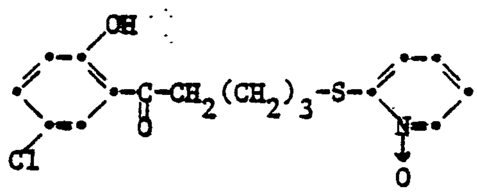

(400)

als bräunliche Substanz mit einem Schmelzpunkt von 205-207°C.

Auf analoge Weise werden unter Verwendung der entsprechenden ω-Chlor-hydroxyacylphenone als Ausgangsmaterialien die in Tabelle 5 angeführten Verbindungen der Formel

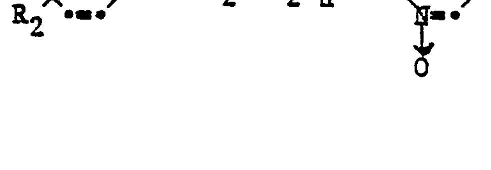

(401)

erhalten.

BAD ORIGINAL

Tabelle 5

| Verbindung Nr. | Stellung OH | $R_1$ | $R_2$ | n | Fp(°C) |
|---|---|---|---|---|---|
| 402 | 4-OH | H | H | 2 | 194-197 |
| 403 | 2-OH | H | 5-Cl | 2 | 130-132 |
| 404 | 2-OH | 4-Cl | 5-Cl | 1 | 207-208 |
| 405 | 2-OH | H | 5-$CH_3$ | 2 | 108-110 |
| 406 | 2-OH | H | 5-F | 2 | 186-189 |
| 407 | 2-OH | H | 5-F | 3 | |
| 408 | 2-OH | H | H | 2 | |
| 409 | 2-OH | 4-Cl | 5-Cl | 2 | |
| 410 | 2-OH | H | H | 3 | |
| 411 | 4-OH | H | H | 3 | |
| 412 | 4-OH | 2-Cl | 3-Cl | 1 | 213-215 |

Die im vorstehenden Beispiel als Ausgangsprodukte benötigten ω-Chlor-2-hydroxyacylphenone werden folgendermassen erhalten:

42,8 g 4-Chloranisol werden in 30 ml Nitroäthan gelöst. Unter Eiskühlung werden portionenweise 53,4 g $AlCl_3$ bei höchstens 35°C eingetragen. Bei einer Temperatur von 10-15°C werden 42,3 g γ-Chlorbuttersäurechlorid zugetropft und danach 18 Stunden bei Raumtemperatur gerührt. Die erhaltene Suspension wird auf Wasser ausgetragen und mit Chloroform extrahiert. Die Chloroformphase wird neutral gewaschen, getrocknet und eingedampft, wobei 69,6 g eines braunen Oels zurückbleiben. Nach 2 Tagen bei 5°C kristallisiert das Oel teilweise. Der entstandene Kristallbrei wird auf einem Tonteller verrieben. Man erhält dabei 53,5 g γ-Chlor-2-hydroxy-5-chlorbutyrophenon der Formel

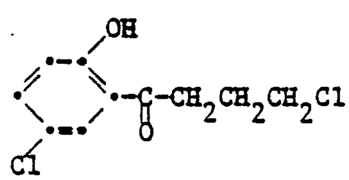 (412)

BAD ORIGINAL

in Form farbloser Kristalle mit einem Schmelzpunkt von 41-42°C.

Auf analoge Weise werden die in Tabelle 6 angeführten ω-Chlor-hydroxyacylphenone der Formel

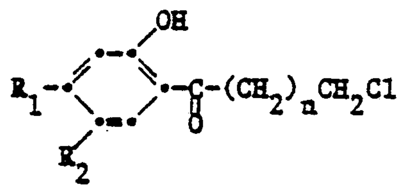

(413)

erhalten.

Tabelle 6

| Verbindung Nr. | $R_1$ | $R_2$ | n | Fp (°C) |
|---|---|---|---|---|
| 414 | H | $CH_3$ | 2 | 39-41 |
| 415 | H | F | 2 | 62-66 |
| 416 | H | F | 3 | |
| 417 | Cl | Cl | 1 | 117-119 |
| 418 | H | Cl | 3 | 70-71 |
| 419 | H | H | 2 | |

Die ebenfalls als Ausgangsprodukte benötigten ω-Chlor-4-hydroxyacylphenone werden nach folgender Vorschrift erhalten:

32,4 g Anisol werden in 30 ml Chlorbenzol gelöst. Unter Eiskühlung werden portionenweise 53,6 g $AlCl_3$ bei höchstens 35°C eingetragen. Bei einer Temperatur von 15-35°C werden 42,3 g γ-Chlorbuttersäurechlorid zugetropft und danach langsam auf 100°C erwärmt. Nach dreistündigem Rühren bei 100°C wird auf Raumtemperatur abgekühlt, auf Wasser ausgetragen und anschliessend mit Aether extrahiert. Durch Eindampfen der neutral gewaschenen und getrockneten Aetherphase wird ein rötlicher Feststoff erhalten. Dieser wird aus Hexan/Aethanol unter Verwendung von Aktivkohle umkristallisiert.

BAD ORIGINAL

Man erhält dann 45 g γ-Chlor-4-hydroxybutyrophenon der Formel

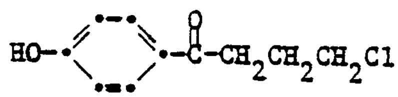

(419)

in Form farbloser Kristalle mit einem Schmelzpunkt von 108-111°C.

Auf analoge Weise erhält man die Verbindung der Formel

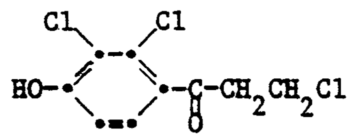

(420)

mit einem Schmelzpunkt von 92-95°C.

Beispiel 5: **Prüfung der bakteriziden und fungiziden Aktivität der Wirkstoffverbindungen der Formeln (100)-(102), (200), (202)-(211), (300), (302)-(307), (400) und (402)-(412) im Suspensionstest:**

Ein Phosphatpuffermedium (pH 5, 7 und 8), das jeweils 1'000, 100, 10, 1 oder 0,1 ppm der zu prüfenden Wirkstoffverbindung enthält, wird mit jeweils einem Teststamm (Bakterien: O/n-Kulturen; Pilze: Sporensuspension, 14-tägige Kulturen) inoculiert (Endkonzentration $10^6$ Keime/ml). Nach einer Inkubationsdauer von 18 Stunden bei 20°C auf einem Magnetrührwerk wird geprüft, bei welcher Konzentration eine Abtötung der Keime eingetreten ist. Alle geprüften Verbindungen zeigen in diesem Test eine ausgezeichnete keimtötende Wirkung.

Als Testkeime werden verwendet:

| | |
|---|---|
| Staph. aureus | ATCC 6538 |
| E. coli | ATCC 11229 |
| Ps. aeruginosa | ATCC 15442 |
| Aspergillus niger | ATCC 6275 |

Medium: Phosphatpuffer nach Sörensen (1/15 molar) mit 2% Brain-Heart-Infusion-Broth.

Beispiel 6: Prüfung der bakteriostatischen und fungistatischen Aktivität der Wirkstoffverbindungen der Formeln (100)-(102), (200), (202)-(211), (300), (302)-(307), (400) und (402)-(412) nach dem Agar-Inkorporationstest:

Von jeder Wirkstoffverbindung wird jeweils eine 5%ige Stammlösung in ® Methylcellosolve (Methylglykol) bereitet. Von dieser Stammlösung wird eine Verdünnungsreihe bereitet, sodass sich die Konzentrationen in den einzelnen Lösungen um jeweils eine Zehnerpotenz unterscheiden. Von den so erhaltenen Lösungen werden jeweils 0,3 ml in sterile Petrischalen vorgelegt und mit jeweils 15 ml heissem, flüssigem Nährmedium (Nutrient-Agar) vermischt. Im Nährmedium sind dann 1'000, 100, 10, 1 und 0,1 ppm an Aktivsubstanz enthalten.

Nach dem Erstarren der Platten werden auf diese mit einer Pasteurpipette bzw. mit dem Impfapparat die jeweiligen Keimsuspensionen (es werden dieselben Keime wie in Beispiel 5 verwendet) aufgetropft. Die Inkubationszeit für Bakterien beträgt 24 Stunden bei 37°C, für Aspergillus niger 3 Tage bei 28°C. Es wird danach beurteilt, bis zu welcher Konzentration an Wirksubstanz die Keime gewachsen sind. Die geprüften Verbindungen zeigen eine gute bakteriostatische bzw. fungistatische Wirkung gegenüber den geprüften Keimen.

Beispiel 7: Die Verbindungen der Formeln (100)-(102), (200), (202)-(211), (300), (302)-(307), (400) und (402)-(412) werden zusammen mit Seife in ein Nährmilieu eingearbeitet und die Wirksamkeit gemäss Agar-Incorporations-Test bestimmt. Als Testkeime werden die folgenden verwendet:

Staph. aureus ATCC 6538
Strept. faecalis ATCC 10541
Corynebacterium minutissimum NCTC 10288
Escherichia coli NCTC 8196
Salmonella typhimurium NCTC 5710

BAD ORIGINAL

Pseudomonas aeruginosa NCTC 8060

Candida albicans ATCC 10259

Trich. mentagrophytes ATCC 9533

Nährboden für Bakterien: Trypton-Glucose-Extrakt-Agar

Nährboden für Pilze: Mycophil-Agar.

Aus einer Grundseifenmasse wird mit sterilem Wasser eine 0,5%ige Lösung hergestellt.

Von dieser Stammlösung wird soviel zu heissem, sterilem, flüssigem Agar gegeben, dass das Nährmedium 500 ppm Seife enthält.

Die Testsubstanzen werden in DMSO gelöst, Gehalt 500 ppm. In sterilen Petrischalen werden 0,1, 0,05 und 0,01 ml Aktivsubstanzlösung vorgelegt und mit 10 ml Nährmedium, enthaltend 500 ppm Seife, versetzt und gut vermischt. (Im Nährmedium sind somit 5, 2,5 und 0,5 ppm Aktivsubstanz vermischt.)

Nach dem Erstarren der Platten werden die Keimsuspensionen mit einer Pasteurpipette resp. mit dem Impfapparat aufgetropft. Die Inkubationszeit für Bakterien beträgt 24 Stunden bei 37°C, für Pilze 5 Tage bei 28°C. Es wird beurteilt, ob die Keime gewachsen sind oder nicht.

Die auf diese Weise geprüften Verbindungen zeigen eine gute Wirksamkeit gegen die verwendeten Mikroorganismen.

BAD ORIGINAL

Beispiel 8: Die Verbindungen der Formeln (100)-(102), (200), (202)-(211), (300), (302)-(307), (400) und (402)-(412) werden in einer geeigneten Formulierung (Aethylcellosolve/Dimethylformamid) gelöst. Die unten aufgeführten drei Substrate werden in die Formulierbäder eingelegt und anschliessend zwischen 2 Aluminiumfolien abgequetscht; die Substrate werden darauf luftgetrocknet. Die Abquetschung wird so durchgeführt, dass sich 1000 ppm Wirksubstanz auf dem Gewebe befinden.

1. Baumwolle renforcé, laugiert, gebleicht
   $m^2$-Gewicht: 121 g
2. Polyamid, Nylon-Stapelgewebe, fixiert, gebleicht
   $m^2$-Gewicht: 140 g
3. Polyester, Dacron-Stapelgewebe, Typ 54, fixiert, gebleicht
   $m^2$-Gewicht: 130 g

Die Substrate werden darauf nach dem Agardiffusionstest (modif. AATCC-Test Methode 90, 1970) gegen die unten aufgeführten 7 Testorganismen geprüft.

Bakterien:

Staphylococcus aureus ATCC 6538

Escherichia coli NCTC 8196

BAD ORIGINAL

Proteus mirabilis NCTC 8309
Pseudomonas aeruginosa NCTC 8060

Fungi:
Candida albicans ATCC 10259
Trichophyton mentagrophytes ATCC
Aspergillus niger ATCC 6275.

Die Testplatten bestehen aus einem Zweischichtenagar, d.h. aus einer Grundschicht aus unbeimpftem Nähragar und einer Deckschicht aus angeimpftem Nähragar.

Bakterien: Nutrient-Agar Fungi: Mycophil-Agar.

Die filtrierte Keimsuspension wird auf eine erstarrte Grundschicht gegossen und nach dem Erstarren der beimpften Schicht werden Rondellen zu 20 mm Durchmesser der behandelten Substrate aufgelegt. Die Bebrütung der Bakterien- und Candida-Platten erfolgt während 24 Stunden bei 37°C, die Fungi-Platten werden während 3 bis 5 Tagen bei 28°C inkubiert. Nach der Inkubation werden die Platten hinsichtlich Hemmzone ausgewertet. Bei fehlender Hemmzone wird der Bewuchs unter dem Prüfling mit der Lupe kontrolliert.

Die auf diese Weise geprüften, oben angeführten Verbindungen zeigen in Verbindung mit den verwendeten Substraten eine gute Wirkung gegen die verwendeten Bakterien und Pilze.

Beispiel 9: Rohpapier, das zu 90% aus gebleichter Sulfitcellulose und 10% aus Birke besteht, wird in der Leimpresse mit einer 2,5%igen Lösung jeweils einer der Verbindungen der Formeln (100)-(102), (200), (202)-(211), (300), (302)-(307), (400) und (402)-(412) in Methanol/Wasser 2:1 so imprägniert, dass die Gewichtszunahme 40% beträgt.

BAD ORIGINAL

Das getrocknete Papier enthält, bezogen auf sein Eigengewicht, 1% Wirkstoff.

Zur Prüfung der Wirkung gegen Bakterien werden Rondellen zu 10 mm Durchmesser des imprägnierten Papiers auf Brain Heart Infusion-Agar-Platten gelegt, die mit Staphylococcus aureus vorbeimpft sind. Die Platten werden hierauf 24 Stunden bei 37°C bebrütet.

Zur Prüfung der Wirkung gegen Pilze werden Rondellen zu 25 mm Durchmesser auf Mycophil-Agar-Platten gelegt und anschliessend mit Aspergillus niger beimpft. Die Platten werden dann 72 Stunden bei 30°C bebrütet.

Beurteilt wird einerseits die um die Papier-Rondellen auftretende Hemmzone (HZ in mm) und andererseits das mikroskopisch feststellbare Wachstum (W in %) unter bzw. auf den Rondellen. Die geprüften Verbindungen zeigen eine gute Wirkung gegen die verwendeten Testkeime.

Beispiel 10: Zur Herstellung einer antimikrobiellen Stückseife werden 2,4 g einer der Verbindungen der Formeln (100)-(102), (200) (202)-(211), (300), (302)-(307), (400) und (402)-(412) folgender Mischung zugesetzt:

120 g Grundseife in Schuppenform

0,12 g Dinatriumsalz der Aethylendiamintetraessigsäure (Dihydrat)

0,24 g Titandioxyd.

Die durch Walzen erhaltenen Seifenspäne werden mit einem Schnellrührer pulverisiert und anschliessend zu Seifenstücken gepresst.

Konzentrierte wässrige Lösungen der antimikrobiellen Seife werden warmem Brain Heart Infusion-Agar so zugemischt, dass Inkorporations-Verdünnungsreihen mit 2, 10, 20, 100 usf. ppm Wirkstoff entstehen. Die warmen Mischungen werden in Petrischalen gegossen, erstarren gelassen und anschliessend mit Staphylococcus aureus beimpft.

Nach 24-stündiger Bebrütung bei 37°C wird die minimale Hemmkonzentration bestimmt.

Die geprüften Verbindungen zeigen eine gute Wirkung gegen die Testkeime.

Beispiel 11: Man bereitet eine Lösung von 1,00 g einer der Verbindungen der Formeln (100)-(102), (200), (202)-(211), (300), (302)-(307), (400) und (402)-(412) und 3,00 g Natrium-sulforicinoleat in 47,00 g Polyäthylenglykol 400 und eine Lösung von 7,00 g Natriumdodecylsulfat in 39,85 g Wasser, mischt die beiden Lösungen und versetzt das Gemisch mit 0,15 g Parfum. Man erhält auf diese Weise ein gut wirksames Händedesinfektionsmittel, das zur Applikation auf die feuchte Haut getropft oder gespritzt und verrieben wird.

Beispiel 12: Durch Mischen der folgenden Bestandteile wird ein emulgierbares Konzentrat hergestellt:

10 Teile einer der Verbindungen der Formeln (100)-(102), (200), (202)-(211), (300), (302)-(307), (400) und (402)-(412)
68 Teile Xylol
10 Teile Dimethylformamid
12 Teile grenzflächenaktive Verbindung.

Das Konzentrat wird vor der Anwendung mit Wasser auf die 50- bis 500-fache Menge verdünnt. In der so erhaltenen Emulsion

BAD ORIGINAL

werden Holz, Sägemehl oder Cellulosefasern getränkt, wodurch sie gegen Bakterien- und Pilzbefall geschützt sind.

Beispiel 13: Durch Mischen der folgenden Bestandteile wird ein öllösliches Konzentrat hergestellt:

20 Teile einer der Verbindungen der Formeln (100)-(102), (200), (202)-(211), (300), (302)-(307), (400) und (402)-(412)
40 Teile Aethylenglykolmonoäthyläther
10 Teile Dimethylformamid
30 Teile Xylol

Das erhaltene Konzentrat wird einer Anstrichflotte bzw. einem Schneidöl derart zugemischt, dass die Farbe bzw. das Oel 0,1% an Wirkstoffverbindung enthält . Es wird dadurch ein Schutz vor Bakterien- und Pilzbefall erreicht.

Beispiel 14: Das gemäss Beispiel 12 hergestellte emulgierbare Konzentrat wird mit Wasser auf das 400- bzw. 800-fache verdünnt.

Quadratische Prüfkörper aus Birkenholz mit einer Seitenlänge von 5 cm und einer Dicke von 5 mm werden 2 Minuten in die Emulsion getaucht und 24 Stunden bei Raumtemperatur getrocknet. Sodann werden die Proben auf die Oberfläche von Agarplatten gelegt. Auf die Agarplatten und die Proben werden Sporensuspensionen von Aspergillus niger gesprüht. Die Pilze werden 2 Wochen bei einer Luftfeuchtigkeit von 95% und einer Temperatur von 28°C inkubiert.

Im Vergleich zu unbehandelten Prüfkörpern wird eine starke Wachstumshemmung festgestellt.

BAD ORIGINAL

Patentansprüche

1. Hydroxyphenylketone der Formel

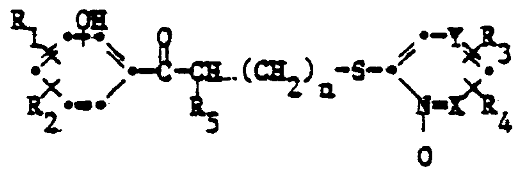,

worin n die Zahl 0, 1, 2 oder 3 darstellt, wobei die Hydroxylgruppe nur dann in 3-Stellung stehen kann, wenn n 0 oder 1 ist, X ein Kohlenstoff- oder Stickstoffatom, Y ein Kohlenstoff- oder Stickstoffatom oder eine Gruppe >N→O bedeuten, wobei der heterocyclische Ring jedoch höchstens 2 Stickstoffatome als Ringglieder enthalten kann, $R_1$ und $R_2$ unabhängig voneinander Wasserstoff, Fluor, Chlor, Brom, Nitro, Methyl oder Methoxy, $R_3$ Wasserstoff, Chlor, Brom, Nitro, Cyano, Methyl, Methoxy, Amino oder $-NHCOCH_3$, $R_4$ Wasserstoff, Chlor, Brom oder Methyl oder $R_3$ und $R_4$ für den Fall, dass X für ein Kohlenstoffatom steht, gemeinsam die Ergänzung zu einem an Bindung [b] des heterocyclischen Ringes anellierten Benzolring und $R_5$ Wasserstoff oder für den Fall, dass n 1 ist, eine Gruppe der Formel

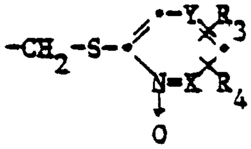

bedeuten, wobei $R_3$, $R_4$, X und Y wie oben definiert sind.

2. Hydroxyphenylketone nach Anspruch 1 der Formel

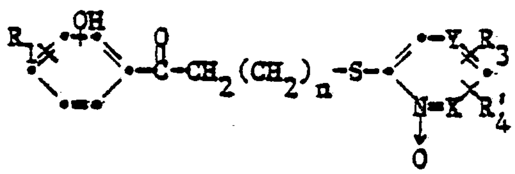

,

worin n, X, Y, $R_1$ und $R_3$ wie in Anspruch 1 definiert sind und $R_4'$ Wasserstoff oder für den Fall, dass X für ein Kohlenstoffatom steht, gemeinsam mit $R_3$ die Ergänzung zu einem an Bindung [b] des heterocyclischen Ringes anellierten Benzolring bedeutet.

3. Hydroxyphenylketone nach Anspruch 2 der Formel

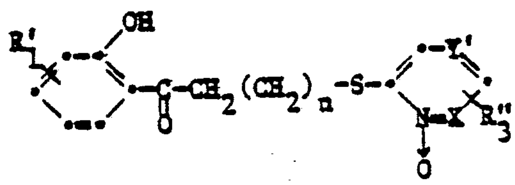

,

worin n die Zahl 0, 1, 2 oder 3, X und Y' unabhängig voneinander ein Kohlenstoffatom oder ein Stickstoffatom, $R_1'$ Wasserstoff, Chlor, Brom, Nitro oder Methyl, vorzugsweise Wasserstoff, Chlor oder Methyl, und $R_3''$ Wasserstoff, Chlor, Brom oder Methyl, vorzugsweise Wasserstoff, Chlor oder Methyl bedeuten.

4. Hydroxyphenylketone nach Anspruch 1 der Formel

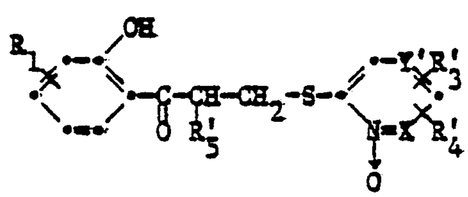

,

worin $R_1$ und X wie in Anspruch 1 definiert sind und Y' ein Kohlenstoff- oder Stickstoffatom, $R_3'$ Wasserstoff, Chlor, Brom oder Methyl, vorzugsweise Wasserstoff, Chlor oder Methyl, und $R_4'$ Wasserstoff oder $R_3'$ und $R_4'$ für den Fall, das X für ein Kohlenstoffatom steht, gemeinsam die Ergänzung zu einem an Bindung [b] des heterocyclischen Ringes

BAD ORIGINAL

anellierten Benzolring, wobei $R_4'$ vorzugsweise für Wasserstoff steht, und $R_5'$ Wasserstoff oder eine Gruppe der Formel

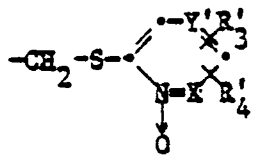

bedeuten, wobei $R_3'$, $R_4'$, X und Y' wie oben definiert sind.

5. Hydroxyphenylketone nach einem der Ansprüche 1 bis 4, worin X und Y bzw. Y' jeweils ein Kohlenstoffatom bedeuten.

6. Hydroxyphenylketone nach Anspruch 2 der Formel

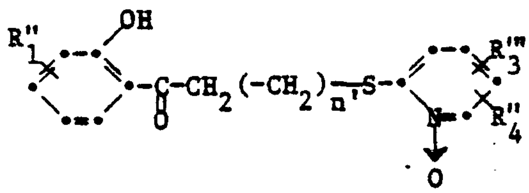 ,

worin $R_1''$, $R_3'''$ und $R_4''$ jeweils unabhängig voneinander Wasserstoff, Chlor oder Methyl und n' 0 oder 1 bedeuten.

7. Verfahren zur Herstellung von Hydroxyphenylketonen der Formel

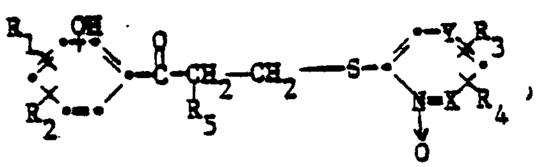 ,

worin X ein Kohlenstoff- oder Stickstoffatom, Y ein Kohlenstoff- oder Stickstoffatom oder eine Gruppe 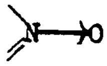 bedeuten, wobei der heterocyclische Ring jedoch höchstens 2 Stickstoffatome als Ringglieder enthalten kann, $R_1$ und $R_2$ unabhängig voneinander Wasserstoff, Fluor, Chlor, Brom, Nitro, Methyl oder Methoxy, $R_3$ Wasserstoff, Chlor, Brom, Nitro, Cyano, Methyl, Methoxy, Amino oder $-NHCOCH_3$, $R_4$ Wasserstoff, Chlor, Brom oder Methyl oder $R_3$ und $R_4$ für den Fall, dass X für ein Kohlenstoffatom steht, gemeinsam die Ergänzung zu einem an Bindung [b] des

heterocyclischen Ringes anellierten Benzolring und $R_5$ Wasserstoff oder eine Gruppe der Formel

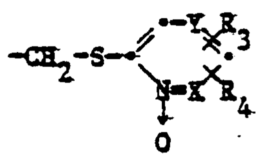

bedeuten, wobei $R_3$, $R_4$, X und Y wie oben definiert sind, dadurch gekennzeichnet, dass man etwa 1 Moläquivalent einer Mannich-Base der Formel

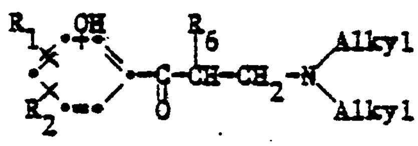 • H Hal ,

worin $R_1$ und $R_2$ wie oben definiert sind und Hal ein Halogenatom und $R_6$ Wasserstoff oder eine Gruppe der Formel

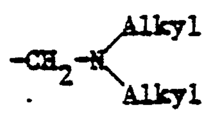 • H Hal

bedeutet, mit etwa einem Moläquivalent (für den Fall, dass $R_6$ Wasserstoff ist) oder mit etwa 2 Moläquivalent (für den Fall, dass $R_6$ nicht Wasserstoff ist) eines Thiols der Formel

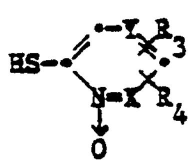

umsetzt, worin X, Y, $R_3$ und $R_4$ wie oben definiert sind.

BAD ORIGINAL

8. Verfahren zur Herstellung von Hydroxyphenylketonen der Formel

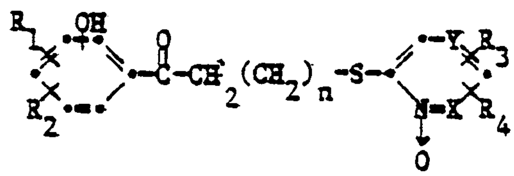

,

worin n die Zahl 0, 1, 2 oder 3 darstellt, wobei die Hydroxylgruppe nur dann in 3-Stellung stehen kann, wenn n 0 ist, X ein Kohlenstoff- oder Stickstoffatom, Y ein Kohlenstoff- oder Stickstoffatom oder eine Gruppe >N→O bedeuten, wobei der heterocyclische Ring jedoch höchstens 2 Stickstoffatome als Ringglieder enthalten kann, $R_1$ und $R_2$ unabhängig voneinander Wasserstoff, Fluor, Chlor, Brom, Nitro, Methyl oder Methoxy, $R_3$ Wasserstoff, Chlor, Brom, Nitro, Cyano, Methyl, Methoxy, Amino oder $-NHCOCH_3$, $R_4$ Wasserstoff, Chlor, Brom oder Methyl oder $R_3$ und $R_4$ für den Fall, dass X für ein Kohlenstoffatom steht, gemeinsam die Ergänzung zu einem an Bindung [b] des heterocyclischen Ringes anellierten Benzolring bedeuten, dadurch gekennzeichnet, dass man eine Verbindung der Formel

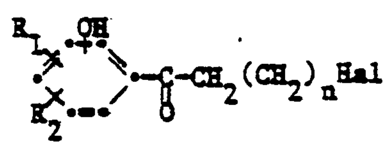

,

worin $R_1$, $R_2$ und n wie oben definiert sind und Hal. Chlor oder Brom bedeutet, wobei Hal nur Brom bedeutet, wenn die Hydroxylgruppe, im Falle n=0, in 3-Stellung steht, mit einem Thiol der Formel

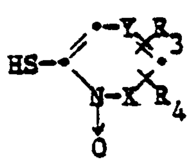

umsetzt, worin X, Y, $R_3$ und $R_4$ wie oben definiert sind.

BAD ORIGINAL

9. Mittel mit antimikrobieller Wirkung, enthaltend ein oder mehrere der im Anspruch 1 definierten Hydroxyphenylketone.

10. Mittel nach Anspruch 9, enthaltend 0,001 bis 10% an Hydroxyphenylketon, bezogen auf das Gesamtmittel, das zusätzlich noch Wasser, Lösungsmittel und/oder übliche Hilfsstoffe wie Netzmittel, Emulgiermittel, Komplexbildner, Duftstoffe, Lichtschutzmittel, Weichmacher, Füllstoffe, weitere antimikrobiell wirksame Verbindungen und/oder Appreturmittel enthalten kann.

11. Mittel nach Anspruch 9 zum Schützen von Holz vor dem Befall durch Bakterien und Pilze, zur Körperpflege mit desinfizierender und desodorierender Wirkung oder zum Schützen von Papiermassen und Apparaturen von Papierfabriken vor durch Mikroorganismen hervorgerufener schleimbildung.

12. Antimikrobielle Seife oder Waschmittel nach Anspruch 9, gekennzeichnet durch einen Gehalt von 0,01 bis 5% an einer im Anspruch 1 definierten Verbindung.

13. Verfahren zur Bekämpfung von Mikroorganismen auf oder in organischen Substraten und zum Schützen dieser Substrate vor Mikroorganismen, dadurch gekennzeichnet, dass man mindestens eine der im Anspruch 1 definierten Verbindungen besagten Substraten einverleibt oder auf deren Oberfläche aufbringt.

14. Verfahren nach Anspruch 13 zum Schützen von nicht-textilen Kunststoffen gegen Befall durch Mikroorganismen, dadurch gekennzeichnet, dass man dem Kunststoff in irgendeiner Phase des Verarbeitungsprozesses oder auch den Monomeren vor der Polymerisation mindestens eine der im Anspruch 1 definierten Verbindungen einverleibt.

BAD ORIGINAL

15. Verfahren nach Anspruch 13 zum Schützen von technischen Formulierungen, insbesondere von Formulierungen von Farbstoffen, Pigmenten, Textilhilfsmitteln, Bindemitteln, Leimen, Bioriden und Anstrichmitteln, vor dem Befall durch Mikroorganismen, dadurch gekennzeichnet, dass man diesen Formulierungen mindestens eine der im Anspruch 1 definierten Verbindungen einverleibt, vorzugsweise in einer Konzentration von 1 bis 10'000 ppm, insbesondere von 10 bis 5000 ppm.

16. Verfahren nach Anspruch 13 zum Schützen von Papier- und Zellstoffmassen sowie von Apparaten in der Papierindustrie vor Schleimbildung hervorrufenden Mikroorganismen, dadurch gekennzeichnet, dass man den Papier- und Zellstoffmassen oder dem Kreislaufwasser mindestens eine der im Anspruch 1 definierten Verbindungen zusetzt, vorzugsweise in einer Konzentration von 1 bis 10'000 ppm, insbesondere 10 bis 5000 ppm.

17. Verfahren nach Anspruch 13 zum Schützen von Holz, Holzspänen und Sägemehl vor dem Befall durch Pilze und Bakterien, vorzugsweise durch Aufbringen von 0,01 bis 10 g mindestens einer im Anspruch 1 definierten Verbindung pro Quadratmeter Holz.

18. Verfahren nach Anspruch 13 zum Schützen von kosmetischen Artikeln, wie Pudern, Shampoos und Salben, vor dem Befall durch Mikroorganismen, dadurch gekennzeichnet, dass man diesen Mitteln in irgend einer Phase ihrer Herstellung mindestens eine der im Anspruch 1 definierten Verbindungen einverleibt.

19. Verfahren nach Anspruch 13 zum Desinfizieren von diversen Oberflächen, z.B. Metall- und Kunststoffoberflächen oder Farbanstrichen oder von Gegenständen im medizinischen Bereich sowie zur Desinfektion der Haut, insbesondere der Hände.

20. Verfahren nach Anspruch 13 zum Entkeimen von Waschgut und zum Schützen von Waschgut gegen Befall durch Mikroorganismen, dadurch gekennzeichnet, dass man Textilien mit Flotten behandelt, die, gegebenenfalls neben waschaktiven Substanzen, mindestens eine der im Anspruch 1 definierten Verbindungen enthalten.

21. Verwendung der im Anspruch 1 definierten Hydroxyphenylketone zur Bekämpfung schädlicher Mikroorganismen und zum Schützen von organischen Substraten vor dem Befall durch Mikroorganismen.

0031795

Nummer der Anmeldung

Europäisches Patentamt

# EUROPÄISCHER RECHERCHENBERICHT

EP 80 81 0392.3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | US - A - 3 772 307 (WARNER-LAMBERT CO.)<br>* Spalten 1, 2 *<br>-- | 1,8,9 |
| D | DE - A - 2 165 752 (L'OREAL)<br>* Ansprüche 1, 16 *<br>-- | 1,9 |
| | US - A - 4 041 033 (COLGATE-PALMOLIVE CO.)<br>* Spalte 1 *<br>-- | 1,9 |
| | DE - A1 - 2 716 832 (UNIROYAL)<br>* Seite 30, Verbindung Nr. 119 *<br>---- | 1 |

KLASSIFIKATION DER ANMELDUNG (Int. Cl.³)

C 07 D 213/89
C 07 D 237/18
A 01 N 43/40
A 01 N 43/58
B 27 K 3/34
C 08 K 5/34
C 11 D 3/28
C 11 D 9/50
D 06 M 13/34

RECHERCHIERTE SACHGEBIETE (Int. Cl.³)

A 01 N 43/00
B 27 K 3/34
C 07 D 213/89
C 07 D 237/18
C 08 K 5/34
C 11 D 3/28
C 11 D 9/50
D 06 M 13/34

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 24-03-1981 | FROELICH |